# EUROPÄISCHE PATENTSCHRIFT

(11) **EP 2 328 473 B1**
(45) Veröffentlichungstag und Bekanntmachung des Hinweises auf die Patenterteilung: **03.03.2021**
(21) Anmeldenummer: 09778130.6
(22) Anmeldetag: 26.08.2009
(51) Int. Cl.: A61B 5/053, A61B 8/00

(54) **ANORDNUNG MIT TASTKOPF MIT WENIGSTENS ZWEI ELEKTRODEN ZUR IMPEDANZMESSUNG UND VERFAHREN HIERZU**
ARRANGEMENT COMPRISING A PROBE HAVING AT LEAST TWO ELECTRODES FOR IMPEDANCE MEASUREMENT, AND METHOD THEREFOR
DISPOSITIF COMPRENANT UNE TÊTE DE DÉTECTION AVEC AU MOINS DEUX ÉLECTRODES SERVANT À MESURER L'IMPÉDANCE ET PROCÉDÉ ASSOCIÉ

(30) Priorität: 27.08.2008 DE 102008039844
(43) Veröffentlichungstag der Anmeldung: 08.06.2011
(73) Patentinhaber: Fresenius Medical Care Deutschland GmbH, 61352 Bad Homburg (DE)
(72) Erfinder: MOISSL, Ulrich, 61184 Karben (DE); CHAMNEY, Paul, Herts HP23 4EW (GB); WABEL, Peter, 61191 Rosbasch (DE)
(74) Vertreter: Bobbert & Partner Patentanwälte PartmbB
(86) Internationale Anmeldenummer: PCT/EP2009/006189
(87) Internationale Veröffentlichungsnummer: WO 2010/022933

(56) Entgegenhaltungen:
- CH-A- 352 781
- DE-C1- 10 136 529
- JP-A- 2000 245 707
- US-A1- 2006 085 049
- US-A1- 2006 085 049
- US-B1- 6 708 060
- US-B2- 7 203 536

## Beschreibung

Die vorliegende Erfindung betrifft eine Anordnung mit einem Bioimpedanzmessgerät und einem Tastkopf mit wenigstens zwei Messelektroden für die Impedanzmessung am Körper eines Patienten. Sie betrifft ein Verfahren zum Messen von Impedanz eines Körpers gemäß dem Oberbegriff des Anspruchs 12

Die Bio-Impedanzmessung bzw. Impedanzmessung am Patienten ist aus der Praxis als ein Verfahren beispielsweise zum Bestimmen der Körperzusammensetzung, insbesondere des Wassergehaltes eines Patienten bekannt. Mittels dieses Verfahrens können beispielsweise Änderungen des Flüssigkeitshaushalts oder des Ernährungszustands sowie Gewebezusammensetzungen eines Patienten erkannt und/ oder differenziert werden, wie dies beispielsweise aus der WO 2006/00285 A1 und der US 6,678,552 B2 bekannt ist.

Bislang erfolgen Impedanzmessungen oder Bio-Impedanzmessungen als Messungen von Summenimpedanzen oder aufsummierten Impedanzänderungen einzelner Körperextremitäten oder Abschnitte hiervon (Arm, Unterschenkel), des Rumpfes oder als Ganzkörpermessung. Bei jedem der zuvor genannten Verfahren wird über Elektroden Strom in den Körper eingeleitet und eine resultierende Spannung auf der Oberfläche gemessen. Aus den Messergebnissen lässt sich die Gewebeimpedanz ermitteln. Die dabei vorgesehenen Elektroden für die Stromeinleitung einerseits und die Elektroden für die Spannungsmessung andererseits werden beispielsweise im Handgelenks- und Sprunggelenksbereich mit entsprechenden Befestigungsbändern am Körper fixiert. Die Elektroden sind im Stand der Technik dabei stets ortsfest platziert.

Lokal erhöhte oder verminderte Impedanzen, wie sie beispielsweise bei einem Ödem des Unterschenkels oder einer lokal begrenzten Flüssigkeitsansammlung, wie beim Aszites, auftreten, fließen dabei in das Ergebnis bspw. einer Ganzkörper-Impedanzmessung ein. Sie bleiben aufgrund ihres oftmals geringen relativen Beitrags zur Summenbildung unentdeckt, können Messergebnisse verfälschen oder können einzelnen Abschnitten des Körpers nicht ausreichend präzise zugeordnet werden. Dasselbe trifft auf lokale Impedanzmessungen zu, die ebenfalls stets mit fest platzierten Elektroden durchgeführt werden.

Aus der US 2006/0085049 A1 sind eine aktive, auf Bioimpedanz basierende Elektrode, ein Gewebediskriminierungssystem sowie Verfahren zu deren Verwendung bekannt.

Aus der JP 200245707 A ist ein Körperfettmesser mit bewegbarer Elektrode zur Messung der Körperimpedanz bekannt.

Aus der CH 352781 sind ein Verfahren und eine Vorrichtung zur Feststellung von Eigenschaften und Verschiedenheiten von Körpern, insbesondere im organischen Aufbau des menschlichen oder tierischen Körpers sowie von zeitlichen Veränderungen desselben bekannt.

Aus der DE 101 36 529 C1 ist ein kombinierter elektrischer Impedanz- und Ultraschallscanner bekannt.

Aus der US 7,203,536 B2 sind ein Verfahren und eine Vorrichtung zur Bestimmung der Körperzusammensetzung durch bioelektrische Impedanzmessungen in Tiefenrichtung bekannt.

Aus der US 6,708,060 B1 sind ein Handapparat und ein Verfahren zur Medikamentenzuführung durch die Haut sowie zur Extraktion von Analyten bekannt.

Aufgabe der vorliegenden Erfindung ist es, eine weitere Ausstattung für die Durchführung einer Impedanzmessung am Patienten und entsprechende Verfahren anzugeben.

Die vorliegende Erfindung wird gelöst mit den Merkmalen des Anspruchs 1.

Erfindungsgemäß wird eine Anordnung mit einem Bioimpedanzmessgerät vorgeschlagen, welche wenigstens einen Tastkopf umfasst, welcher wiederum zwei Elektroden, von welchen wenigstens eine als eine Messelektrode, insbesondere als Spannungsmesselektrode, ausgestaltet ist, für die Impedanzmessung am Körper eines Patienten aufweist. Die Anordnung umfasst ferner wenigstens eine weitere Elektrode, insbesondere eine Stromeinleitungselektrode. Außerdem weist der Tastkopf eine Einrichtung zum Bestimmen einer Geschwindigkeit, mit welcher der Tastkopf über die Oberfläche des Patienten bewegt wird, und/oder zum Bestimmen einer vom Tastkopf zurückgelegten Wegdifferenz, jeweils auf oder über der Oberfläche des Körpers des Patienten, auf.

Unter einem Tastkopf wird erfindungsgemäß eine Vorrichtung mit den oben genannten wenigstens zwei Elektroden verstanden, welche zum Messen oder Bestimmen eines elektrischen Feldes, einer Spannung, der Impedanz und/oder Änderungen jeweils hiervon oder zum Einleiten von Strom im wesentlichen frei - d. h. ortsunabhängig - über den Körper eines Patienten bewegbar ist. Dabei kann die Art der freien Bewegbarkeit jener eines vom Arzt eingesetzten Ultraschallkopfes entsprechen. Der Tastkopf kann dabei in dem für die Untersuchung erforderlichen Umfang bewegbar, mittels Leitungen an einer Untersuchungseinheit, angebunden sein. Jedoch kann anstelle der Leitungen auch eine körperlose Übertragung mit einer Untersuchungseinheit erfolgen. In diesem Fall können Leitungen zwischen Tastkopf und Untersuchungseinheit entbehrlich sein.

Die Messelektroden oder die Stromeinleitungselektroden des Tastkopfes erlauben eine Bestimmung oder Messung, insbesondere eine Spannungsbestimmung oder - messung, an dem mittels des Tastkopfes untersuchten Oberflächenbereich des mit einer Leistungsquelle verbundenen Patienten. Bewegt der Benutzer des Tastkopfes diesen von einem ersten Bereich auf der Körperoberfläche zu einem zweiten Bereich, so misst der Tastkopf zunächst die Impedanz des ersten Bereichs und dann jene des zweiten Bereichs. Dabei kann sowohl der erste Bereich als auch der zweite Bereich zwischen zwei Stromeinleitungselektroden liegen. Der Tastkopf ist somit dergestalt, dass seine Elektroden eben nicht fest platziert am Körper verbleiben, wie dies beim Stand der Technik der Fall ist. Der Tastkopf ist im Gegensatz hierzu ortsunabhängig am Körper des untersuchten Patienten einsetzbar. Ganz allgemein ist erfindungsgemäß unter "ortsunabhängig" zu verstehen, dass eine Untersuchung von mehr als nur einem Körperbereich ohne Lösen und gegebenenfalls erneutem Festlegen von Elektroden wie Stromeinleitungselektroden oder Messelektroden am untersuchten Körper mittels Klebeverbindung, Klettband, Gummizug oder dergleichen erfolgen kann.

Der Begriff des "Patienten" trifft im Rahmen der vorliegenden Erfindung sowohl auf Mensch als auch auf Tier zu. Ferner kann die vorliegende Erfindung zur Impedanzmessung an lebendem Gewebe sowie post mortem für die Untersuchung toten Gewebes durch den Pathologen oder Gerichtsmediziner eingesetzt werden.

Vorteilhafte Weiterbildungen dieser vorliegenden Erfindung sind jeweils Gegenstand der Unteransprüche.

So wird in einer bevorzugten Ausführungsform vorgeschlagen, den Tastkopf derart auszugestalten, dass wenigstens zwei der Elektroden des Tastkopfes in Kontakt mit demselben Körperabschnitt des Patienten in Berührung bringbar sind. Dabei können die betreffenden Elektroden zugleich bzw. gleichzeitig z. B. auf der Haut des Bauchs, der Brust oder in einem Gelenkbereich des Patienten als Beispiele "desselben Körperabschnitts" zur Impedanzmessung, insbesondere auch zwischen beiden Elektroden, aufgesetzt werden. Als derselbe Körperabschnitt kann erfindungsgemäß ein Bereich verstanden werden, in welchem Hautareale liegen, welche im Gebrauch des Tastkopfes jeweils Kontakt zu einer der Elektroden haben, welche in der anatomischen Neutral-Null-Stellung nur wenige Zentimeter voneinander entfernt liegen. Von "demselben Körperbereich" wird erfindungsgemäß daher nicht gesprochen, wenn beispielsweise eine Elektrode Kontakt (ein solcher Kontakt ist hier stets als elektrischer Kontakt zu verstehen) mit der Haut des Oberbauchs des Patienten hat, eine weitere Elektrode hingegen Kontakt mit der Haut im Handbereich des Patienten oder gar einer weiteren Person hat. Eine Elektrode, die während der Untersuchung des Patienten mit dem Tastkopf Kontakt mit der Haut der Hand hat, wird erfindungsgemäß als Handelektrode bezeichnet, wie sie aus dem Stand der Technik bekannt ist, nicht aber als Tastkopfelektrode im Sinne der Erfindung.

In einer weiter bevorzugten Ausführungsform der Erfindung wird vorgeschlagen, wenigstens zwei der Elektroden des Tastkopfes mit einem festen Abstand zueinander anzuordnen. Dieser Abstand kann beispielsweise fünf oder zehn Zentimeter betragen, wobei auch andere Werte, beispielsweise solche zwischen einem Zentimeter und fünfzehn Zentimetern, von der Erfindung umfasst sind. Letztendlich steigt die räumliche Auflösung, desto dichter die Elektroden angeordnet sind. Andererseits sinkt dann die Signalstärke. Die Entfernung der Elektroden ist daher an der Anwendung zu orientieren.

In einer besonders vorteilhaften Ausführungsform können die beiden Messelektroden relativ zueinander bewegbar und ggf. arretierbar sein. Dabei kann der Abstand von wenigstens zwei Elektroden des Tastkopfes zueinander variabel oder verstellbar sein. Dies kann beispielsweise mittels einer Verschiebeeinrichtung für wenigstens eine Elektrode realisiert sein.

In einer wiederum weiter bevorzugten Ausführungsform der vorliegenden Erfindung weist der Tastkopf wenigstens eine Einrichtung zum Bestimmen einer Position des Tastkopfes auf oder über der Oberfläche des Körpers des Patienten auf.

Dabei kann die Einrichtung zum Bestimmen einer Position des Tastkopfes am Tastkopf selbst vorgesehen sein. Sie kann erfindungsgemäß aber auch anstelle (oder ergänzend) am Tastkopf an einer den Tastkopf enthaltenden, ebenfalls von der vorliegenden Erfindung umfassten Anordnung, vorgesehen sein.

Die Bestimmung, welche Position der Tastkopf bei einer konkreten Impedanzmessung, bezogen auf die Körperoberfläche oder einen Referenzpunkt, einnimmt, erlaubt einen Vergleich der bei dieser konkreten Impedanzmessung gemessenen Werte mit Werten einer Referenzpopulation an der gleichen Körperstelle. Zudem erlaubt sie vergleichende, reproduzierbare Messungen zu verschiedenen Zeitpunkten an demselben Patienten. Letzteres ermöglicht es vorteilhaft, Veränderungen der Impedanz und damit des untersuchten Gewebes über der Zeit mit bislang nicht erzielbarer Präzision, insbesondere eine räumliche Auflösung oder Zuordnung betreffend, festzustellen.

Die genaue Kenntnis der Position des Tastkopfes während der Impedanzmessung ermöglicht vorteilhaft eine vereinfachte Kombination der mittels Impedanzmessung und anderen Verfahren wie Ultraschall erzielten Ergebnisse, welche bei Kenntnis der jeweiligen Position zu aussagekräftigen, dreidimensionalen Darstellungen zusammenführbar sind. Auch sind die Erstellung von impedanz-tomographischen Darstellungen, vor allem bei Einsatz mehrerer Tastköpfe, bei genauer Kenntnis der jeweiligen Position des Tastkopfes/der Tastköpfe bei der Messung vorteilhaft möglich.

Über Funksensoren kann die Position des Tastkopfes relativ zu beispielsweise Hand-, Arm- und Beinelektroden als Referenzpunkte ständig automatisch erfasst werden. Das System ist damit in der Lage, selbstständig zu erkennen, dass beispielsweise eine Messung im Bereich der Leber durchgeführt wird. Eine automatische Abgleichung mit Referenzwerten von Gesunden durch Vergleich der gemessenen Impedanz mit Werten vergleichbarer Oberflächenbereiche von Referenzpersonen können Abweichungen der Impedanz in jenem Körperbereich aufdecken.

In einer wiederum weiter bevorzugten Ausführungsform der vorliegenden Erfindung bestimmt die Einrichtung zum Bestimmen einer Wegdifferenz die mit dem Tastkopf zwischen der ersten und der zweiten Position auf der Körperoberfläche oder bezogen auf die Körperoberfläche oder einen beliebigen Referenzpunkt zurückgelegte Wegdifferenz.

Der bei der Untersuchung des Patienten mittels des Tastkopfes zurückgelegte Weg kann beispielsweise über ein Rollrad erfasst werden. Ebenso ist eine Erfassung der Position des Tastkopfes (auch unabhängig von einem zurückgelegten Weg) mittels optischem Korrelations-System oder mittels Funksensoren zum Feststellen der Position von der vorliegenden Erfindung umfasst.

Dabei können die zusätzlichen Elektroden, welche beispielsweise als Stromeinleitungselektroden ausgestaltet sind, wie auch im Stand der Technik üblich, den Strom beispielsweise im Bereich des Handgelenks oder des Sprunggelenks ein- bzw. ausleiten oder dort messen. Auch eine Anordnung von Fuß zu Fuß, Kopf zu Fuß, Kopf zu Hand oder Hand zu Hand ist selbstverständlich denkbar.

Die vorliegende Erfindung wird ferner gelöst durch ein Verfahren gemäß Ansprüch12. Da die hiermit jeweils erzielbaren Vorteile ungeschmälert denjenigen entsprechen, wie sie auch mit der Anordnung des Anspruchs 1 erzielbar sind, wird zur Vermeidung von Wiederholungen an dieser Stelle ausdrücklich auf deren oben stehende Diskussion verwiesen.

Der Tastkopf kann mittels mobiler Leistungsquelle - bspw. batteriebetrieben - oder gespeist von einer festen Energieeinrichtung - wie z. B. jener der verwendeten Untersuchungseinheit - verwendet werden.

Die Elektroden des Tastkopfes - nicht aber die zusätzlichen Elektroden - können erfindungsgemäß auch auf mehr als nur einen Tastkopf verteilt vorgesehen sein. Dies erlaubt dann beispielsweise eine Messung von Bauch zu Rücken oder umgekehrt, sowie die Untersuchung der Impedanz über größere, dennoch variabel bestimmbare Abstände. Unter einem Tastkopf wird erfindungsgemäß daher auch ein mehrteiliger Tastkopf, bei welchem einzelne Teile mit jeweils wenigstens einer Elektrode, insbesondere einer Messelektrode, getrennt voneinander verwendet werden können, verstanden. Unter Tastkopf kann erfindungsgemäß auch eine Mehrzahl von Tastköpfen verstanden werden.

Die Bio-Impedanz bzw. Impedanz (beide Begriffe sind hier gegeneinander austauschbar zu verstehen) kann mittels des Tastkopfes an einer Frequenz (Bio-Impedanzanalyse) gemessen werden. Es kann jedoch auch eine Spektroskopie zur Bestimmung einer Ortskurve durchgeführt werden.

Mittels des Tastkopfes kann vorteilhaft bspw. gezielt die Herzpulsation mittels Impedanzmessung, beispielsweise an einem der großen arteriellen Gefäße, gemessen oder erfasst werden. Ebenso kann die Einlagerung von Wasser in den Lungen vorteilhaft erfasst werden. Beides sind Messungen, die bedingt durch die stets ortsfeste Platzierung der Elektroden im Stand der Technik nicht möglich sind.

Eine Unterscheidung von Fettgewebe in subkutanes Fett oder viscerales Fett ist mittels des Tastkopfes aufgrund seiner lokalen Aufsetzbarkeit bzw. der lokal erfolgenden Messung der Impedanz vorteilhaft möglich. Auch ein Links-Rechts-Vergleich ist gezielt durchführbar. Der Tastkopf eignet sich ferner zum Einsatz in der Rehabilitation eines Patienten beim Muskelaufbau, beispielsweise nach einer Gelenkoperation. Er eignet sich zum Erkennen von Lymphödemen und kann sowohl im Liegen als auch im Stehen eingesetzt werden. Eine Kombination seines Einsatzes mit dem Wiegen des Patienten ist ebenfalls erfindungsgemäß möglich. Damit können die mittels des Tastkopfes erzielten Resultate die Impedanz betreffend in Bio-Impedanz-Modellgleichungen, bei welchen das Gewicht ebenfalls eine Rolle spielt, einfließen, um gesundheitsrelevante Aussagen zu erzielen.

Durch streifendes oder rollendes Bewegen des Tastkopfes am Körper, beispielsweise vom Hals hinunter bis zum Fuß, kann eine Impedanzverteilung entlang des Weges erstellt und ggf. aufgezeichnet werden.

Mittels des Tastkopfes ist es möglich, schnell und auf einfache Weise unterschiedliche Bereiche des Körpers des Patienten abzutasten und auf auffällige Impedanzwerte oder -änderungen zu untersuchen. Auf diese Weise können selbst nur lokal vorliegende Ödeme detektiert und eventuell in ihrem Umfang quantifiziert werden.

Zum Erzielen eines guten Elektrodenkontakts zwischen Tastkopf und Oberfläche des Patienten kann ein Leitmittel, bspw. ein leitfähiges Gel, eingesetzt werden, wie dies z. B. von Ultraschalluntersuchungen bekannt ist.

Der Vergleich mit Referenzdaten für verschiedene, abgeleitete Messwerte der Bio-Impedanz, wie beispielsweise Muskelmasse, Fettmasse und Wassereinlagerung, ist denkbar. Dies betrifft nicht nur die Impedanz-Rohdaten.

Die Messung kann als Spektroskopie mit einer Vielzahl von Frequenzen oder monofrequent bei beispielsweise 50 kHz durchgeführt werden. Des Weiteren können die Ergebnisse mit anderen bildgebenden Verfahren, wie beispielsweise MRT (Kernspin) oder DEXA (Dual-Energy-XRay-Absorptiometry) kombiniert werden. Entsprechende Rechen-, Speicher- und/oder Anzeige- oder Ausgabeeinrichtungen können vorgesehen sein. Auch eine gleichzeitige Ableitung von EKG-Signalen oder Teilen hiervon über die, zum Zwecke der Impedanzmessung bereits angelegten oder ortsunabhängig eingesetzten, Elektroden ist möglich. Dabei können lokale Bioimpedanzsignale direkt mit EKG-Signalen korreliert werden (z. B. Detektion von Extrasystolen). Entsprechende Rechen-, Speicher- und/oder Anzeige- oder Ausgabeeinrichtungen können auch hierfür vorgesehen sein.

Ferner ist es möglich, eine Standard-Skinfold-Messung zur Beurteilung des Fettgehalts mit einer Impedanzmessung mittels des Tastkopfes und/oder Systems zu kombinieren. Hierbei würde eine Spannungsmessung zwischen den Skinfold-Klemmen erfolgen. Wiederum können entsprechende Rechen-, Speicher- und/oder Anzeige- oder Ausgabeeinrichtungen hierfür vorgesehen sein.

Mittels des Tastkopfes, der Anordnung sowie der Verfahren kann vorteilhaft eine lokale Impedanzmessung auch zwischen den beiden Elektroden vorteilhaft möglich sein. Letzteres kann eine hochselektive Messung über einem abgegrenzten Hautbereich bedeuten oder ermöglichen.

Zur Ausführung des erfindungsgemäßen Verfahrens können die erforderlichen Einrichtungen und Mittel vorgesehen sein. Diese können zum jeweiligen Zweck des erfindungsgemäßen Verfahrens konfiguriert sein.

Vorzugsweise weist die Anordnung eine Einrichtung auf, welche konfiguriert ist zum Wiedergeben von Ergebnissen bildgebender Untersuchungen unter Berücksichtigung von Ergebnissen einer Ultraschalluntersuchung des Patienten. Diese Einrichtung kann eine Recheneinheit und/oder eine Einheit zum Darstellen der Ergebnisse sein, z. B. ein Monitor, ein Drucker, eine Datenbank, usw.

Vorzugsweise weist die Anordnung eine Einrichtung auf, welche konfiguriert ist zum Wiedergeben von Ergebnissen bildgebender Untersuchungen des Patienten unter Erstellen von impedanztomografischen Ergebnissen. Diese Einrichtung kann ebenfalls eine Recheneinheit und/oder eine Einheit zum Darstellen der Ergebnisse sein, z. B. ein Monitor, ein Drucker, eine Datenbank, usw.

Die vorliegende Erfindung wird im Folgenden mit Bezug auf die beigefügte, schematisch vereinfachte Zeichnung exemplarisch detaillierter erläutert. In der Zeichnung gilt:
- Fig. 1: zeigt in Fig. 1a die herkömmliche Durchführung einer Impedanzmessung am Körper eines Patienten sowie das erfinderische Verfahren unter Einsatz eines Tastkopfes gemäß der vorliegenden Erfindung in Fig. 1b;
- Fig. 2: zeigt eine mögliche Ausführung des Tastkopfes gemäß der vorliegenden Erfindung in einer Seitenansicht;
- Fig. 3: zeigt schematisch vereinfacht eine Messanordnung für die Impedanzmessung oder für ein Verfahren zum Ableiten von Spannungsdifferenzen auf der Körperoberfläche eines Patienten gemäß der vorliegenden Erfindung;
- Fig. 4: zeigt beispielhafte, mittels der Anordnung der Fig. 3 mögliche Spannungsableitungen;
- Fig. 5: zeigt wie mittels eines Abrollens eines nicht dargestellten Rollrades eine Wegdifferenz über das Abdomen des Patienten hinweg ermittelbar ist; und
- Fig. 6: zeigt schematisch vereinfacht eine erfindungsgemäße Anordnung.

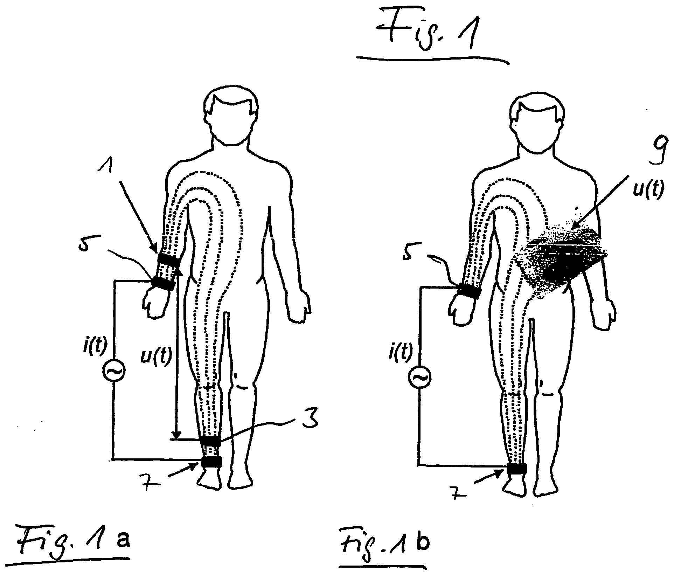

Fig. 1 zeigt in Fig. 1a eine Elektrodenanordnung und einen Stromfluss bei einer herkömmlich durchgeführten Ganzkörper-Bio-Impedanzmessung, wie sie beispielsweise mit dem Fresenius-Body-Composition Monitor durchgeführt werden kann. Dabei werden dem Patienten sowohl im Bereich des Handgelenks als auch im Bereich des Sprunggelenks Spannungsmesselektroden 1, 3 angelegt. Mittels der Spannungsmesselektroden 1, 3 wird die, durch einen über Stromeinleitungselektroden 5, 7 in den Körper eingeleiteten Strom induzierte, Spannung gemessen. Lokal erhöhte oder verminderte Impedanzen, wie sie bei dem oben exemplarisch genannten Beinödem oder dem Aszites auftreten können, werden bei dieser Messanordnung nicht zuverlässig erkannt. Ferner ist eine Zuordnung eines die Impedanz verändernden Ereignisses oder Zustandes nicht dem betroffenen Abschnitt des Körpers des Patienten zuzuordnen.

Fig. 1b zeigt die Impedanzmessung gemäß der vorliegenden Erfindung. Dabei wird die über die Stromeinleitungselektroden 5, 7 induzierte Spannung lokal mittels eines wenigstens zwei (nicht dargestellte) Spannungsmesselektroden aufweisenden Tastkopfes 9 gemessen. Die Impedanz kann daraufhin, wie auch im Stand der Technik, auf einfache Weise errechnet werden. Die Spannungselektroden, welche in Fig. 1b nicht dargestellt sind, sind im Tastkopf 9 implementiert. Sie können dabei beispielsweise einen festen Abstand von einem bis zehn oder mehr Zentimeter aufweisen.

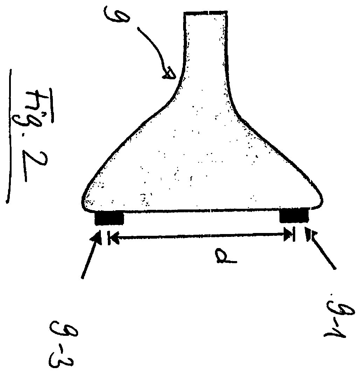

Fig. 2 zeigt eine mögliche Ausführung des Tastkopfes 9 gemäß der vorliegenden Erfindung in einer Seitenansicht mit Spannungsmesselektroden 9-1, 9-3. Diese haben im Beispiel der Fig. 2 einen Abstand d von fünf Zentimetern zueinander.

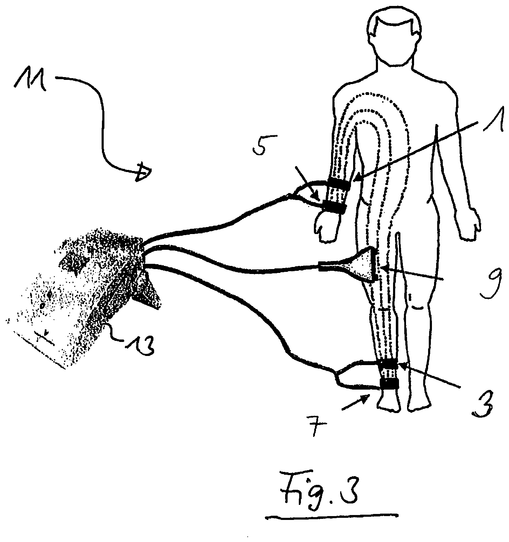

Fig. 3 zeigt schematisch vereinfacht eine Messanordnung 11 für die Impedanzmessung oder für das Verfahren zum Ableiten von Spannungsdifferenzen auf der Körperoberfläche eines Patienten gemäß der vorliegenden Erfindung.

Die Anordung 11 weist in Fig. 3 einen Tastkopf 9, welcher auch als Impedanzfühler oder -sensor bezeichnet wird, Stromeinleitungselektroden 5, 7, wie sie auch in Fig. 1 gezeigt sind, und ein Impedanzmessgerät 13 auf. Das Impedanzmessgerät 13 ist dabei sowohl mit dem Tastkopf 9 als auch mit den Stromeinleitungselektroden 5 und 7 verbunden.

Die Anordnung 11 weist in der in Fig. 3 gezeigten speziellen Ausführungsform zum Ermöglichen einer Spannungs- bzw. Impedanzermittlung neben den Messelektroden des Tastkörpers 9 noch zusätzliche Spannungselektroden 1 und 3 auf. Diese Elektroden 1 und 3 sind hierbei rein optional und zur Ausführung der vorliegenden Erfindung nicht erforderlich. Sie stellen lediglich eine mögliche, von der vorliegenden Erfindung ebenfalls umfasste, Ergänzung dar.

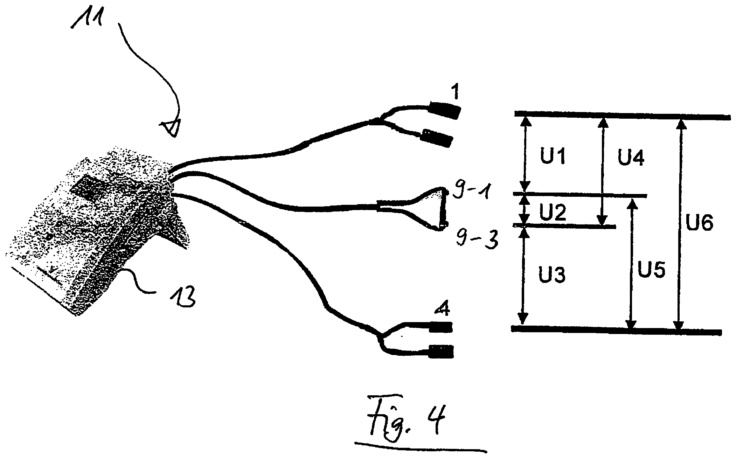

Fig. 4 zeigt exemplarische, mittels der Anordnung 11 der Fig. 3 mögliche Spannungsableitungen. Aufgrund des Vorsehens von insgesamt vier Spannungsmesselektroden 1, 3, 9-1 und 9-3 sind sechs verschiedene Messungen möglich. Dies hat den Vorteil, dass drei unabhängige Körpersegmente aufgespannt werden können (U1, U2 und U3). Darüber hinaus ergibt sich durch Ausnutzung von Redundanz über U4, U5 und U6 eine Möglichkeit zum vorteilhaften Verringern von Messfehlern. Letzteres ergibt sich aus der Erkenntnis, dass U1 + U2 + U3 gleich U6 sein muss.

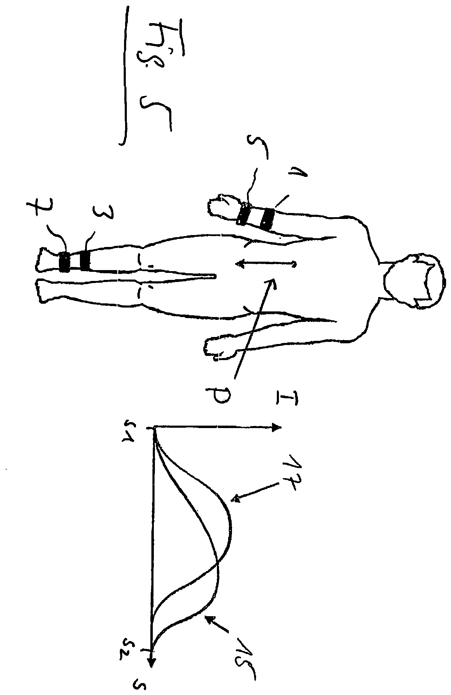

Fig. 5 zeigt, wie mittels Abrollens eines nicht dargestellten Rollrades als Beispiel einer Einrichtung zum Bestimmen einer Wegdifferenz über das Abdomen des Patienten hinweg eine Impedanzkurve 15 aufgezeichnet werden kann. Diese kann mit einer Impedanzkurve 17 einer Normpopulation verglichen werden. Bei entsprechender Abweichung, beispielsweise der durch die Kurve aufgezeigten Fläche oder der Extremwerte, beispielsweise des Maximums, der Impedanzkurve 15 kann auf kritische viszerale Fettmassen oder auf Wassereinlagerungen geschlossen werden.

Die Kurven 15 und 17 der Fig. 5 stellen dabei die Höhe der Impedanz I über dem Weg s dar. Dabei steht s₁ für den Beginn, s₂ für das Ende der Abrollbewegung P mit dem Rollrad über dem Abdomen.

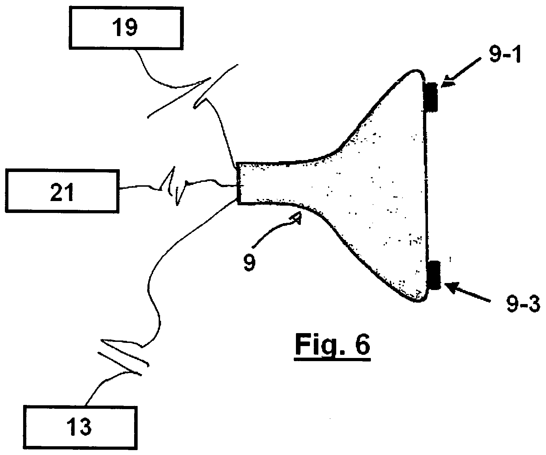

Fig. 6 zeigt schematisch vereinfacht eine erfindungsgemäße Anordnung. Sie weist optional eine oder mehrere Einrichtungen auf, die in Fig. 6 dargestellt sind als ein Bioimpedanzmessgerät 13, als eine Einrichtung 19 zum Wiedergeben von Ergebnissen bildgebender Untersuchungen unter Berücksichtigung von Ergebnissen einer Ultraschalluntersuchung des Patienten, und als eine Einrichtung 21 zum Wiedergeben von Ergebnissen bildgebender Untersuchungen des Patienten unter Erstellen von impedanztomografischen Ergebnissen. Wie in Fig. 6 angedeutet, ist der Tastkopf 9 jeweils direkt oder indirekt mit einer oder allen der Einrichtungen 13, 19 und 21 verbunden.

## Patentansprüche

1. Anordnung (11) mit einem Bioimpedanzmessgerät (13), wenigstens einem Tastkopf (9), welcher wenigstens zwei Elektroden aufweist, von welchen wenigstens eine als Messelektrode, insbesondere als Spannungsmesselektrode (9-1, 9-3), ausgestaltet ist, für die Impedanzmessung am Körper oder an einem Körperabschnitt eines Patienten; und wobei die Anordnung (9) wenigstens eine weitere Elektrode, insbesondere eine Stromeinleitungselektrode (5, 7), aufweist, **dadurch gekennzeichnet, dass** der Tastkopf (9) ferner zur Aufzeichnung einer Impedanzkurve eine Einrichtung zum Bestimmen einer Geschwindigkeit, mit welcher der Tastkopf (9) über die Oberfläche des Patienten bewegt wird, und/ oder zum Bestimmen einer vom Tastkopf (9) zurückgelegten Wegdifferenz (P), jeweils auf oder über der Oberfläche des Körpers des Patienten, aufweist.

2. Anordnung (11) nach Anspruch 1, wobei wenigstens zwei der Elektroden (9-1, 9-3) des Tastkopfs (9) derart am Tastkopf (9) angeordnet sind, dass sie bei dessen Gebrauch gleichzeitig in Kontakt mit demselben Körperabschnitt des Patienten bringbar sind.

3. Anordnung (11) nach Anspruch 1 oder 2, mit festem Abstand zwischen zwei der Elektroden (9-1, 9-3) des Tastkopfs (9).

4. Anordnung (11) nach Anspruch 1 oder 2, wobei der Tastkopf (9) eine Einrichtung zum Verändern des Abstandes von wenigstens zwei der Elektroden (9-1, 9-3) zueinander aufweist.

5. Anordnung (11) nach Anspruch 4, wobei die Einrichtung zum Verändern des Abstandes als eine Einrichtung des Tastkopfs (9) zum Verschieben wenigstens einer der Elektroden (9-1, 9-3) relativ zum Tastkopf (9) und/ oder relativ zu der weiteren Elektrode ausgestaltet ist.

6. Anordnung (11) nach einem der vorangegangenen Ansprüche, wobei der Tastkopf (9) eine Einrichtung zum Bestimmen einer Position (s1, s2) des Tastkopfes (9) auf der Oberfläche des Körpers des Patienten aufweist.

7. Anordnung (11) nach einem der vorangegangenen Ansprüche, wobei die Einrichtung zum Bestimmen einer Wegdifferenz (P) des Tastkopfs (9) als Rollrad, als optisches Korrelations-System, mittels Funksensoren oder als Kombination hiervon ausgestaltet ist.

8. Anordnung (11) nach einem der vorangegangenen Ansprüche , wobei die Stromeinleitungselektroden (5, 7) mit Messelektroden, insbesondere Spannungsmesselektroden (1, 3), verbunden sind.

9. Anordnung (11) nach einem der vorangegangenen Ansprüche, welche eine Einrichtung zum Vergleichen von Messwerten mit bereits vorliegenden Werten aufweist.

10. Anordnung (11) nach einem der vorangegangenen Ansprüche, welche eine Einrichtung (19) aufweist, welche konfiguriert ist zum Wiedergeben von Ergebnissen bildgebender Untersuchungen unter Berücksichtigung von Ergebnissen einer Ultraschalluntersuchung des Patienten.

11. Anordnung (11) nach einem der vorangegangenen Ansprüche, welche eine Einrichtung (21) aufweist, welche konfiguriert ist zum Wiedergeben von Ergebnissen bildgebender Untersuchungen des Patienten unter Erstellen von impedanztomografischen Ergebnissen.

12. Verfahren zum Messen oder Bestimmen von Impedanz, oder Änderungen hiervon, eines Körpers oder Körperteils eines Patienten, zur Aufzeichnung einer Impedanzkurve, **gekennzeichnet durch** den Schritt streifendes oder rollendes Bewegen eines Tastkopfs (9) einer Anordnung gemäß einem der Ansprüche 1 bis 11 am Körper eines Patienten zum Ermitteln einer Impedanzverteilung entlang des Weges.

13. Verfahren nach Anspruch 12 **gekennzeichnet durch** den Schritt Wiedergeben von Ergebnissen bildgebender Untersuchungen unter Berücksichtigung von Ergebnissen einer Ultraschalluntersuchung des Patienten.

14. Verfahren nach einem der Ansprüche 12 bis 13 **gekennzeichnet durch** den Schritt Wiedergeben von Ergebnissen bildgebender Untersuchungen des Patienten unter Erstellen von impedanztomografischen Ergebnissen.

## Claims

1. An arrangement (11) with a bio-impedance measuring apparatus (13), at least one scanning head (9), comprising at least two electrodes, at least one of which is configured as a measurement electrode, in particular as a voltage measurement electrode (9-1, 9-3), for impedance measurement on the body or on a body section of a patient; and wherein the arrangement (11) comprises at least one further electrode, in particular a current introduction electrode (5, 7), **characterized in that**, for recording an impedance curve, the scanning head (9) further comprises a device for determining a speed at which the scanning head (9) is moved over the patient's surface, and/or for determining a differential distance (P) covered by the scanning head (9), in each case, on or above the surface of the patient's body.

2. The arrangement (11) according to claim 1, wherein at least two of the electrodes (9-1, 9-3) of the scanning head (9) are arranged on the scanning head (9) in such a way that, in use, they can be brought simultaneously into contact with the same section of the patient's body.

3. The arrangement (11) according to claim 1 or 2, having a fixed distance between two of the electrodes (9-1, 9-3) of the scanning head (9).

4. The arrangement (11) according to claim 1 or 2, wherein the scanning head (9) comprises a device for changing the distance between at least two of the electrodes (9-1, 9-3).

5. The arrangement (11) according to claim 4, wherein the device for changing the distance is configured as a device of the scanning head (9) for moving at least one of the electrodes (9-1, 9-3) relative to the scanning head (9) and/or relative to the other electrode.

6. The arrangement (11) according to anyone of the preceding claims, wherein the scanning head (9) comprises a device for determining a position (s1, s2) of the scanning head (9) on the surface of the patient's body.

7. The arrangement (11) according to anyone of the preceding claims, wherein the device for determining a differential distance (P) of the scanning head (9) is configured as a roller wheel, as an optical correlation system, by means of radio sensors, or as a combination thereof.

8. The arrangement (11) according to anyone of the preceding claims, wherein the current introduction electrodes (5, 7) are connected to measurement electrodes, in particular voltage measurement electrodes (1, 3).

9. The arrangement (11) according to anyone of the preceding claims, which comprises a device for comparing measured values with already available values.

10. The arrangement (11) according to anyone of the preceding claims, which comprises a device (19) configured to display results of medical imaging examinations taking into account results of an ultrasound examination of the patient.

11. The arrangement (11) according to anyone of the preceding claims, which comprises a device (21) configured to display results of medical imaging examinations of the patient by generating impedance-tomographic results.

12. A method for measuring or determining the impedance, or changes thereof, of a body or body section of a patient, for recording an impedance curve, **characterized by** the step of:
- rubbing or rolling movement of a scanning head (9) of an arrangement according to anyone of claims 1 to 11 over the body of a patient in order to determine an impedance distribution along the path.

13. The method according to claim 12, **characterized by** the step of:
- displaying results of medical imaging examinations taking into account results of an ultrasound examination of the patient.

14. The method according to anyone of claims 12 to 13, **characterized by** the step of:
- displaying results of medical imaging examinations of the patient by generating impedance-tomographic results.

## Revendications

1. Un agencement (11) équipé d'un appareil de mesure de bio-impédance (13), d'au moins une tête de balayage (9) comprenant au moins deux électrodes, dont au moins l'une est conçue comme électrode de mesure, notamment comme électrode de mesure de tension (9-1, 9-3), pour la mesure d'impédance sur le corps ou sur une partie du corps d'un patient; et où l'agencement (11) comprend au moins une autre électrode, notamment une électrode d'introduction de courant (5, 7), **caractérisé en ce que**, pour l'enregistrement d'une courbe d'impédance, la tête de balayage (9) comprend en outre un dispositif permettant de déterminer une vitesse à laquelle la tête de balayage (9) est déplacée sur la surface du patient, et/ou permettant de déterminer une différence de distance (P) parcourue par la tête de balayage (9), dans chaque cas, sur ou au-dessus de la surface du corps du patient.

2. L'agencement (11) selon la première revendication, où au moins deux des électrodes (9-1, 9-3) de la tête de balayage (9) sont disposées sur la tête de balayage (9) de sorte que, lors de leur utilisation, elles puissent être mises en contact simultanément avec la même partie du corps du patient.

3. L'agencement (11) selon la revendication 1 ou 2, ayant une distance fixe entre deux des électrodes (9-1, 9-3) de la tête de balayage (9).

4. L'agencement (11) selon la revendication 1 ou 2, où la tête de balayage (9) comprend un dispositif permettant de modifier la distance entre au moins deux des électrodes (9-1, 9-3).

5. L'agencement (11) selon la revendication 4, où le dispositif permettant de modifier la distance est conçu comme dispositif de la tête de balayage (9) pour déplacer au moins l'une des électrodes (9-1, 9-3) par rapport à la tête de balayage (9) et/ou par rapport à l'autre électrode.

6. L'agencement (11) selon l'une quelconque des revendications précédentes, où la tête de balayage (9) comprend un dispositif permettant de déterminer une position (s1, s2) de la tête de balayage (9) sur la surface du corps du patient.

7. L'agencement (11) selon l'une quelconque des revendications précédentes, où le dispositif permettant de déterminer une différence de distance (P) de la tête de balayage (9) est conçu comme roue de roulement, système de corrélation optique, au moyen de capteurs radio ou d'une combinaison de ceux-ci.

8. L'agencement (11) selon l'une quelconque des revendications précédentes, où les électrodes d'introduction de courant (5, 7) sont reliées à des électrodes de mesure, notamment à des électrodes de mesure de tension (1, 3).

9. L'agencement (11) selon l'une quelconque des revendications précédentes, qui comprend un dispositif permettant de comparer des valeurs mesurées avec des valeurs déjà disponibles.

10. L'agencement (11) selon l'une quelconque des revendications précédentes, qui comprend un dispositif (19) configuré pour reproduire des résultats d'examens d'imagerie médicale en tenant compte des résultats d'un examen échographique d'un patient.

11. L'agencement (11) selon l'une quelconque des revendications précédentes, qui comprend un dispositif (21) configuré pour reproduire des résultats d'examens d'imagerie médicale du patient tout en produisant des résultats tomographiques d'impédance.

12. Un procédé permettant de mesurer ou de déterminer l' impédance, ou des modifications de celle-ci, d'un corps ou d'une partie du corps d'un patient, pour l'enregistrement d'une courbe d'impédance, **caractérisé par** l'étape consistant à:
- déplacer une tête de balayage (9) d'un agencement selon l'une quelconque des revendications 1 à 11, en la frôlant ou en la faisant rouler sur le corps d'un patient afin de déterminer une répartition d'impédance le long de la trajectoire.

13. Le procédé selon la revendication 12, **caractérisé par** l'étape consistant à:
- reproduire des résultats d'examens d'imagerie médicale en tenant compte des résultats d'un examen échographique du patient.

14. Le procédé selon l'une des revendications 12 à 13, **caractérisé par** l'étape consistant à:
- reproduire des résultats d'examens d'imagerie médicale du patient tout en produisant des résultats tomographiques d'impédance.
